(19) Europäisches Patentamt
European Patent Office
Office européen des brevets

(11) Veröffentlichungsnummer: **0 183 217**
**B1**

(12) # EUROPÄISCHE PATENTSCHRIFT

(45) Veröffentlichungstag der Patentschrift: **13.06.90**

(21) Anmeldenummer: **85114930.2**

(22) Anmeldetag: **25.11.85**

(51) Int. Cl.[5]: **C 07 D 207/22,**
C 07 D 207/34,
C 07 C 255/24, A 01 N 43/36

(54) **Mikrobizide Mittel.**

(30) Priorität: **28.11.84 CH 5677/84**

(43) Veröffentlichungstag der Anmeldung:
**04.06.86 Patentblatt 86/23**

(45) Bekanntmachung des Hinweises auf die Patenterteilung:
**13.06.90 Patentblatt 90/24**

(84) Benannte Vertragsstaaten:
**AT BE CH DE FR GB IT LI LU NL**

(56) Entgegenhaltungen:
**AT-B- 308 082**
**CH-A- 480 332**
**DE-A-2 927 480**

(73) Patentinhaber: **CIBA-GEIGY AG**
**Klybeckstrasse 141**
**CH-4002 Basel (CH)**

(72) Erfinder: **Martin, Pierre, Dr.**
**Meisenweg 38**
**CH-4310 Rheinfelden (CH)**

(74) Vertreter: **Zumstein, Fritz, Dr. et al**
**Bräuhausstrasse 4**
**D-8000 München 2 (DE)**

**EP 0 183 217 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft neue 3-Cyano-4-phenylpyrrolin-Derivate mit mikrobizider Wirksamkeit, weiterhin Mittel, die diese Wirkstoffe als Aktivsubstanzen enthalten sowie die Verwendung der Wirkstoffe oder der sie enthaltenden Mittel zur Bekämpfung schädlicher Mikroorganismen, insbesondere pflanzenschädigender Pilze und Bakterien. Die Erfindung betrifft ferner die Herstellung der neuen Verbindungen.

Die neuen Verbindungen entsprechen der allgemeinen Formel I

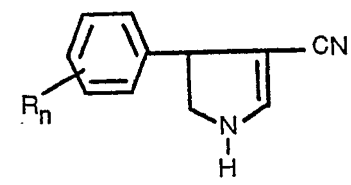

(I),

in welcher

R Halogen, $C_1$—$C_6$-Alkyl oder $C_1$—$C_6$-Haloalkyl darstellt und n 0, 1 oder 2 bedeutet.

Unter Alkyl selbst sowie als Bestandteil von Haloalkyl sind beispielsweise folgende geradkettige oder verzweigte Gruppen zu verstehen: Methyl, Ethyl, Propyl, Butyl, Pentyl oder Hexyl sowie deren Isomere wie z.B. Isopropyl, Isobutyl, sek.-Butyl, tert.-Butyl, Isopentyl, Neopentyl oder Isopentyl.

Halo als Bestandteil des Substituenten Haloalkyl bedeutet das einfache oder mehrfache Auftreten von Fluor, Chlor, Brom oder Jod, vorzugsweise Fluor, Chlor oder Brom, im Alkylrest. Solche halogenierten Alkyle sind beispielsweise: $CH_2Cl$, $CH_2F$, $CHCl_2$, $CHF_2$, $CH_2CH_2Br$, $C_2Cl_5$, $CH_2Br$ oder $CHBrCl$, vorzugsweise $CF_3$.

Bevorzugt sind Verbindungen der Formel I, in denen R Chlor, Brom oder Methyl darstellt und n 1 oder 2 bedeutet.

Insbesondere bevorzugt sind Verbindungen der Formel I, in denen R Chlor oder Methyl und n 2 bedeuten.

Bevorzugte Einzelverbindung ist 3-Cyano-4-(2′,3′-dichlorphenyl)-$\Delta^2$-pyrrolin.

Die Verbindungen der Formel I werden erfindungsgemäss hergestellt, indem Verbindungen der Formel II

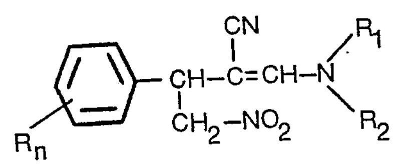

(II)

zu Verbindungen der Formel III

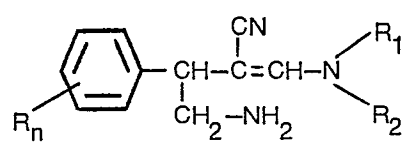

(III)

in welchen $R_1$ und $R_2$ unabhängig voneinander $C_1$—$C_4$-Alkyl, oder zusammen mit dem benachbarten Stickstoffatom Pyrrolidinyl oder Piperidinyl oder mit einem zusätzlichen Sauerstoffatom Morpholinyl bedeuten und $R_n$ die unter Formel I angegebene Bedeutung hat, reduziert und anschliessend zu Verbindungen der Formel I zyklisiert werden.

Eine bevorzugte Ausführungsform des Verfahrens liegt vor, wenn $R_1$ und $R_2$ Methyl bedeuten.

Die Reduktion findet in organischen Lösungsmitteln bei erhöhten Temperaturen unter Wasserstoffdruck in Gegenwart eines Katalysators statt.

Für den Ringschluss sind erhöhte Temperaturen und gegebenenfalls das Vorhandensein eines schwach sauren Milieus erforderlich.

Als Reaktionsmedium kommen folgende Lösungsmittel in Betracht:

Aliphatische und aromatische Kohlenwasserstoffe, wie z.B. Benzol, Toluol, die Xylole oder Petrolether; Ester, wie z.B. Essigsäureäthylester, Essigsäurepropylester oder Essigsäurebutylester; Ether und etherartige Verbindungen, z.B. Dialkylether wie Diethylether, Diisopropylether, tert.-Butylmethylether oder z.B. Anisol und insbesondere cyclische Ether, wie z.B. Dioxan oder Tetrahydrofuran; Alkohole, z.B. Alkanole wie z.B. Methanol oder Ethanol.

Ferner kommen in Betracht Gemische derartiger Lösungsmittel.

Das erfindungsgemässe Verfahren findet bei Temperaturen von 0° bis 150°C statt. Der bevorzugte

Bereich liegt für die Reduktion zwischen 15° und 110°C. Der Ringschluss wird vorzugsweise bei Temperaturen von 50° bis 150°C durchgeführt.

Die Reduktion wird in allgemeinen auf katalytischem Wege durchgeführt. Für diesen Zweck geeignete Katalysatoren sind beispielsweise aus Platin, Palladium, Rhodium, Cobaltpolysulfid oder Raney-Nickel. Bevorzugt sind dabei Katalysatoren aus Platin-Aktivkohle und Cobaltpolysulfid.

Die für den Reduktionsvorgang verwendeten Wasserstoffdrucke liegen im allgemeinen zwischen 1 bar bis 150 bar Anfangsdruck, gemessen bei Raumtemperatur. Bevorzugt für die Reduktion sind Wasserstoffdrucke von 2 bis 120 bar.

Vorteilhafterweise kann im vorhergehend beschriebenen Verfahren zur Herstellung der Verbindungen der Formel I die Reduktion der Verbindungen der Formel II und die Zyklisierung der reduzierten Verbindungen der Formel III in einem einzigen Reaktionsschritt durchgeführt werden. Diese Reaktionsweise stellt eine bevorzugte Ausführungsform des erfindungsgemässen Verfahrens dar. Die Reaktionstemperaturen betragen dabei 15°—150°C.

Die für die erfindungsgemässe Synthese als Zwischenprodukte verwendeten Verbindungen der Formel II sind neu. Sie werden hergestellt, indem man Verbindungen der Formel IV

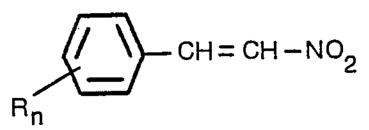

(IV),

mit Verbindungen der Formel V

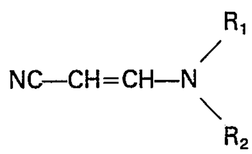

(V),

in welchen $R_1$ und $R_2$ unabhängig voneinander $C_1$—$C_4$-Alkyl, vorzugsweise Methyl, oder zusammen mit dem benachbarten Stickstoffatom Pyrrolidinyl, Piperidinyl oder mit einem zusätzlichen O-Atom Morpholinyl bedeuten und R und n die unter Formel I angegebenen Bedeutungen besitzen, bei erhöhter Temperatur in inerten Lösungsmitteln reagieren lässt.

Als Lösungsmittel kommen in Betracht aliphatische und aromatische Kohlenwasserstoffe, wie z.B. Benzol, Toluol, die Xylole oder Petroläther, vorzugsweise Toluol; ferner Aether wie z.B. Dioxan oder Tetrahydrofuran, insbesondere Tetrahydrofuran; sowie Alkohole wie z.B. Methanol oder Ethanol, insbesondere Ethanol. Ferner kommen in Betracht Gemische derartiger Lösungsmittel sowie Gemische der Lösungsmittel mit Wasser.

Das Verfahren findet bei Temperaturen von 0° bis 200°C, vorzugsweise von 40° bis 130°C statt.

Die als Ausgangsmaterial für die vorhergehende Reaktion dienende Verbindung der Formel IV ist ebenfalls neu. Sie kann beispielsweise hergestellt werden, indem man Verbindungen der Formel VI

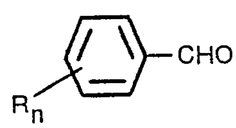

(VI),

in welcher R und n die unter Formel I angegebenen Bedeutungen besitzen, mit Nitromethan in Gegenwart von Ammoniumazetat in Eisessig bei Temperaturen von 70° bis 130°C umsetzt.

Die Benzaldehyde der Formel VI sind allgemein bekannt und können nach bekannten Verfahren hergestellt werden.

Die neuen Verbindungen der Formel

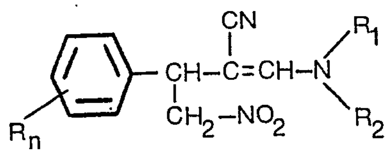

(II)

in welcher $R_1$ und $R_2$ unabhängig voneinander $C_1$—$C_4$-Alkyl, vorzugsweise Methyl, oder zusammen mit dem benachbarten Stickstoffatom Pyrrolidinyl oder Piperidinyl oder mit einem zusätzlichen Sauerstoffatom Morpholinyl bedeuten, stellen wertvolle Zwischenprodukte für die erfindungsgemässe Herstellung der neuen mikrobizid aktiven Verbindungen der Formel I dar und bilden somit einen Bestandteil der vorliegenden Erfindung.

Ein weiterer Gegenstand vorliegender Erfindung betrifft die Herstellung von Verbindungen der Formel VII

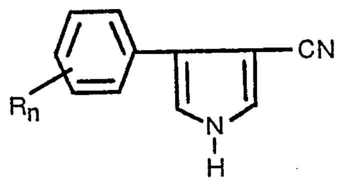

(VII),

in welcher $R_n$ die unter Formel I angegebenen Bedeutungen besitzt, die durch Oxidation der neuen Verbindungen der Formel I herstellbar sind. Die Oxidation wird mittels Brom oder in Gegenwart eines als oxidierendes Medium wirkenden Katalysators in inerten Lösungsmitteln bei Temperaturen von 0° bis 250°C durchgeführt.

Bei Verwendung von Brom sind Temperaturen von 0° bis 80°C und bei Einsatz eines Katalysators Temperaturen von 150° bis 250°C bevorzugt. Als Katalysatoren kommen solche für Oxidationsreaktionen (Wasserstoffübertragung) geeignete in Betracht, wobei Palladium-Aktivkohle bevorzugt ist.

Es kommen als Katalysatoren auch mehrwertige Metallkationen in Frage. Technisch sehr vorteilhafte Kationen sind $Cu^{++}$ und besonders $Fe^{+++}$. Als Salze sind insbesondere $FeCl_3$ oder $FeBr_3$ bevorzugt.

Zur Oxidation wird Luft oder Sauerstoff durch das Reaktionsgemisch geleitet oder im Autoklaven unter Druck (z.B 2 bis 120 bar) aufgepresst. Als Lösungsmittel eignen sich Wasser oder wässrige Lösungsmittel-Gemische mit Alkoholen (Methanol, Ethanol, Isopropanol etc.); Dioxan, Tetrahydrofuran; Dimethylsulfoxid; Dimethylformamid oder anderen mit Wasser mischbaren Lösungsmitteln. Der Temperaturbereich bei der Luftoxidation in Gegenwart von Metallsalzen beträgt in der Regel 0°C bis 90°C, bevorzugt 5° bis 80°C.

Verbindungen der Formel VII sind teilweise bekannt, z.B. aus der DE-Offenlegungsschrift 29 27 480. Es wird dort gesagt, dass sie pflanzenfungizide Eigenschaften besitzen.

Die erfindungsgemässen neuen Pyrrolinderivate der Formel I bilden eine wertvolle Bereicherung des Standes der Technik. So konnte überraschend festgestellt werden, dass die Verbindungen der Formel I ein für Freiland-Bedürfnisse sehr günstiges Mikrobizid-Spektrum gegen phytopathogene Pilze und Bakterien aufweisen. Sie können jedoch nicht nur im Ackerbau oder ähnlichen Einsatzgebieten zur Bekämpfung schädlicher Mikroorganismen an Kulturpflanzen, sondern zusätzlich im Vorratsschutz zur Konservierung verderblich Waren eingesetzt werden. Verbindungen der Formel I besitzen sehr vorteilhafte kurative, systemische und insbesondere präventive Eigenschaften und lassen sich zum Schutz von zahlreichen, insbesondere Freilandkulturen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzpflanzenkulturen die auftretenden Mikroorganismen eingedämmt oder vernichtet werden, wobei auch später zuwachsende Pflanzenteile von derartigen Mikroorganismen verschont bleiben.

Die Wirkstoffe sind beispielsweise gegen die den folgenden Klassen angehörenden phytopathogenen Pilze wirksam: Ascomycetes z.B. Erysiphe, Sclerotinia, Fusarium, Monilinia, Helminthosporium; Basidiomycetes wie z.B. Puccinia, Tilletia, Rhizoctonia; sowie die der Klasse der Phytomycetes angehörenden Oomycetes wie Phytophthora. Als Pflanzenschutzmittel können die Verbindungen der Formel I mit besonders gutem Erfolg gegen wichtige Schadpilze aus der Familie der Fungi imperfecti eingesetzt werden, so z.B. gegen Cercospora, Piricularia und vor allem gegen Botrytis. Botrytis spp. (B. cinera, B. allii) stellen mit der Grauschimmelfäule an Reben, Erdbeeren, Aepfeln, Zwiebeln und anderen Obst- und Gemüsesorten einen bedeutenden wirtschaftlichen Schadfaktor dar. Darüber hinaus lassen sich einige Verbindungen der Formel I erfolgreich zum Schutz verderblicher Waren pflanzlicher oder tierischer Herkunft einsetzen. Sie bekämpfen Schimmelpilze wie Penicillium, Aspergillus, Rhizopus, Fusarium, Helminthosporium, Nigrospora und Alternaria sowie Bakterien wie Buttersäurebakterien und Hefen wie Candida.

Als Pflanzenschutzmittel besitzen die Verbindungen der Formel I ein für die praktische Anwendung in der Landwirtschaft sehr günstiges Wirkungsspektrum zum Schutz von Kulturpflanzen, ohne diese durch unerwünschte Nebenwirkungen nachteilig zu beeinflussen.

Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen und an Vorräten pflanzlicher oder tierischer Herkunft.

Darüber hinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen oder Vorratsgütern, das sich durch Applikation der Verbindungen der Formel I bzw. der neuen Mittel auf die Pflanzen, Pflanzenteile, deren Standort oder das Substrat kennzeichnet.

Als Zielkulturen für den Pflanzenschutz gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüssen); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten; (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Als Vorratsschutzmittel werden die Verbindungen der Formel I in unveränderter Form oder vorzugsweise zusammen mit den in der Formulierungstechnik üblichen Hilfsmitteln eingesetzt und werden z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Verstreuen, Bestreichen oder Giessen und die Form des Mittels werden den angestrebten Zielen und den gegebenen Verhältnissen angepasst. Günstige Aufwandmengen liegen im allgemeinen bei 0,01 bis höchstens 2 kg Aktivsubstanz pro 100 kg zu schützendes Substrat; sie hängen jedoch wesentlich von der Beschaffenheit (Grösse der Oberfläche, Konsistenz, Feuchtigkeitsgehalt) des Substrats und dessen Umgebungseinflüssen ab.

Unter Läger- und Vorratsgütern sollen im Rahmen vorliegender Erfindung pflanzliche und/oder tierische Naturstoffe und deren Weiterverarbeitungsprodukte verstanden werden, beispielsweise die nachfolgend aufgezählten und aus dem natürlichen Lebenszyklus herausgenommenen Pflanzen sowie deren Pflanzenteile (Stengel, Blätter, Knollen, Samen, Früchte, Körner), die im frisch geernteten Zustand oder in weiterverarbeitbarer Form vorliegen (vorgetrocknet, befeuchtet, zerkleinert, gemahlen, geröstet). Als Beispiele, die keinen das Anwendungsgebiet limitierenden Charakter im Rahmen dieser Erfindung besitzen, seien folgende Ertragsgüter genannt: Getreide (wie Weizen, Gerste, Roggen, Hafer, Reis, Sorghum und Verwandte); Rüben (wie Möhren, Zucker- und Futterrüben); Kern-, Stein- und Beerenobst (wie Aepfel, Birnen, Pflaumen, Pfirsiche, Mandeln, Kirschen, Erd-, Him- und Brombeeren); Hülsenfrüchte (wie Bohnen, Linsen, Erbsen, Soja); Oelkulturen (wie Raps, Senf, Mohn, Oliven, Sonnenblumen, Kokos, Rizinus, Kakao, Erdnüsse); Gurkengewächse (wie Kürbis; Gurken, Melonen); Fasergewächse (wie Baumwolle, Flachs, Hanf, Jute, Nessel); Citrusfrüchte; Gemüsesorten (wie Spinat, Salat, Spargel, Kohlarten), Zwiebeln, Tomaten, Kartoffeln, Paprika): Lorbeergewächse (wie Avocadom, Cinnamonum, Kampfer) oder Mais, Tabak, Nüsse, Kaffee, Zuckerrohr, Tee, Weintrauben, Kastanien, Hopfen, Bananen, Gras und Heu.

Als Naturprodukte tierischer Herkunft seien insbesondere getrocknete Fleisch- und Fischverarbeitungsprodukte wie Trockenfleisch, Trockenfisch, Fleischkonzentrate, Knochenmehl, Fischmehl und Tiertrockenfutter genannt.

Das behandelte Vorratsgut wird durch Behandlung mit Verbindungen der Formel I nachhaltig vor dem Befall durch Schimmelpilze und andere unerwünschte Mikroorganismen geschützt. Dadurch wird die Bildung toxischer und zum Teil karzinogener Schimmelpilze (Aflatoxine und Ochratoxine) unterbunden, das Gut vor dem Verderben bewahrt und dessen Qualität für längere Zeit aufrechterhalten. Das erfindungsgemässe Verfahren kann auf alle trockenen und feuchten Vorrats- und Lagergüter angewendet werden, die gegen Mikroorganismen, wie Hefen, Bakterien und insbesondere Schimmelpilze anfällig sind.

Eine bevorzugtes Verfahren zum Aufbringen des Wirkstoffes besteht in einem Besprühen oder Benetzen des Substrats mit einer flüssigen Aufbereitung oder im Vermischen des Substrats mit einer festen Aufbereitung der Aktivsubstanz. Das beschriebene Konservierungs-Verfahren ist ein Teil der vorliegenden Erfindung.

Wirkstoffe der Formel I werden üblicherweise in Form von Zusammensetzungen verwendet und können gleichzeitig oder nacheinander mit weiteren Wirkstoffen auf die zu behandelnde Flächen oder Pflanzen gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das Mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt z.B. in Form von Granulat (Bodenapplikation). Die Verbindungen der Formel I können aber auch

auf Samenkörner aufgebracht werden (Coating), indem man die Körner entweder in einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Besonder vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z.B. Phosphatidylethanolamin, Phosphatidylserin, Phosphatidylglycerin, Lysolecithin, Plasmalogene oder Cardiolipin, die man beispielsweise aus tierischen oder pflanzlichen Zellen, insbesondere aus Hirn, Herz, Leber, Eidotter oder Sojabohnen gewinnen kann. Verwendbare Handelsmischungen sind z.B. Phosphatidylchlorin-Mischungen. Synthetische Phospholipide sind z.B. Dioctanoylphosphatidylchlolin und Dipalmitoylphophatidylcholin.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$—$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyltaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. Das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8—22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4—14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykol-

äthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid. Auf dem Vorratsschutzgebiet werden die für die menschliche und tierische Ernährung unbedenklichen Zuschlagstoffe bevorzugt.

Die in der Formulierungstechnik gebräuchlichen Tenside sind u.a. in folgenden Publikationen beschrieben: "McCutcheon's Detergents and Emulsifiers Annual" MC Publishing Corp., Ridgewood New Jersey, 1981;
Helmut Stache "Tensid-Taschenbuch" Carl Hanser-Verlag München/Wien 1981.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Herstellungsbeispiele:

Beispiel 1:
Herstellung von 1-(2',3'-Dichlorphenyl)-2-nitroäthylen

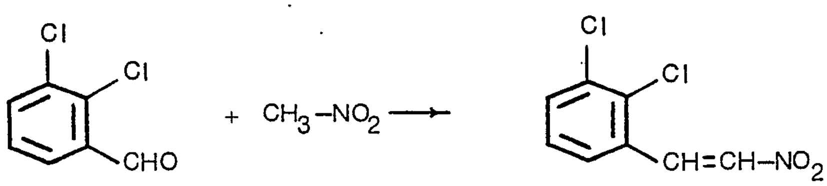

28,0 g 2,3-Dichlorbenzaldehyd, 13,9 g Ammoniumacetat, 13,9 ml Nitromethan und 120 ml Eisessig werden 2 Stunden unter Rückfluss erhitzt. Nach dem Abkühlen wird das Reaktionsgemisch auf Eis gegossen und 30 Minuten gerührt. Der gebildete Niederschlag wird dann abfiltriert, mit Wasser gewaschen und unter Vakuum getrocknet. Es werden schwach gelbliche Kristalle gewonnen.

Smp. 89—91°C. IR ($CHCl_3$) in $cm^{-1}$: 1650 (C=C); 1530 und 1350 ($NO_2$). NMR ($CDCl_3$) in ppm: 7,2—7,7 (m, 4H); 8,50 (d, J=15 Hz, 1H).

Beispiel 2:
Herstellung von 1-Dimethylamino-2-cyano-3-(2',3'-dichlorphenyl)-5-nitro-but-1-en

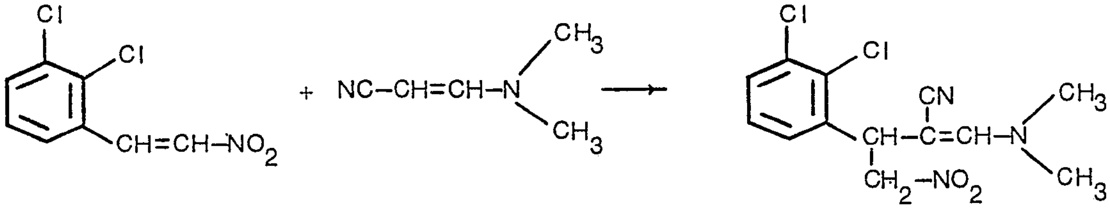

Eine Lösung von 109 g 1-(2',3'-Dichlorphenyl)-2-nitroäthylen, 49 g 1-Cyan-2-dimethylamino-äthylen und 1,0 Liter abs. Toluol wird 50 Stunden unter Rückfluss erhitzt. Nach dem Aküblen wird das Toluol abgedampft und der erhaltene Rückstand mit Diäthyläther gerührt. Anschliessend wird der Niederschlag abfiltriert und mit wenig Diäthyläther nachgewaschen. Es werden beige-farbene Kristalle erhalten.

Smp. 114—115°C. IR ($CHCl_3$) in $cm^{-1}$: 2180 (CN), 1635 (C=C), 1555 ($NO_2$).

NMR ($CDCl_3$) in ppm: 3,08 (s, 6H); 4,6—5,0 (ABX, 3H); 6,60 (s, 1H); 7,2—7,45 (ABX, 3H).

Beispiel 3:
Herstellung von 1-Morpholino-2-cyano-3-(2',3'-dichlorphenyl)-4-nitro-but-1-en

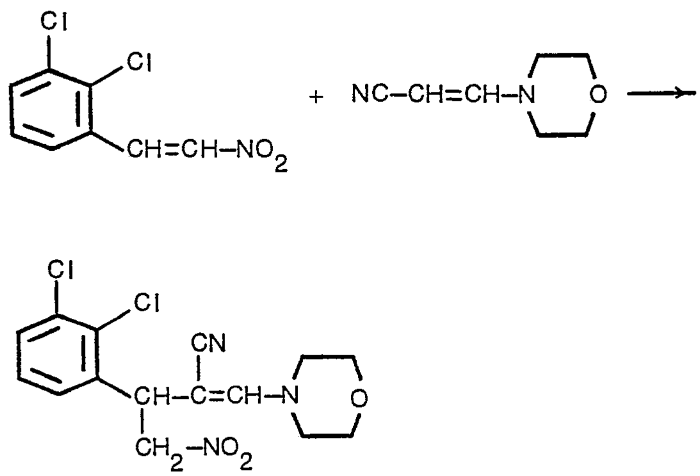

5,0 g 1-(2',3'-Dichlorphenyl)-2-nitroäthylen, 3,18 g 3-Morpholinoacrylnitril und 30 ml Tetrahydrofuran (abs.) werden 24 Stunden unter Rückfluss erhitzt. Anschliessend wird die Reaktionslösung eingedampft. Der erhaltene Rückstand wird in Toluol/Essigsäureäthylester (2:1 V/V) gelöst über Kieselgel chromatographiert. Aus dem Eluat wird das hergestellte Produkt erhalten.

Smp. 74—77°C. IR ($CHCl_3$) in $cm^{-1}$; 2200 (CN), 1630 (C=C), 1560 ($NO_2$).

NMR ($CDCl_3$) in ppm: 4,5 (m, 4H); 4,7 (m, 4H); 4,8 (ABX, 3H); 6,60 (s, 1H); 7,2—7,5 (m, 3H).

Beispiel 4:
Herstellung von 3-Cyano-4-(2',3'-dichlorphenyl)-$\Delta^2$-pyrrolin

a)

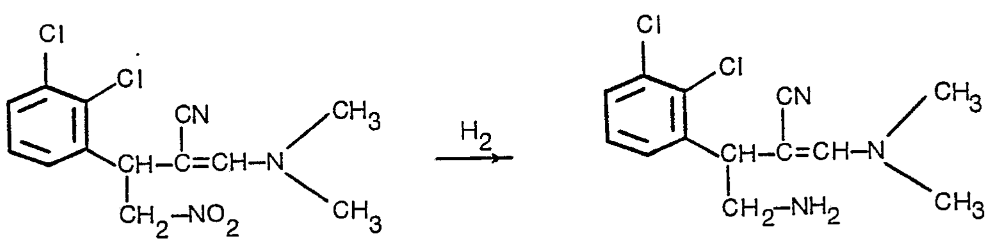


b)

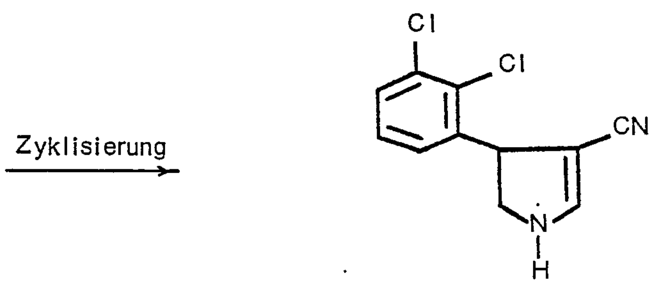


a) 6,28 g 1-Dimethylamino-2-cyano-3-(2',3'-dichlorphenyl)-4-nitrobut-1-en werden in 100 ml Essigsäureäthylester bei 35° und 4 bar in Gegenwart von 1,2 g Platin-Aktivkohle-Katalysator (5 %ig) bis zum Stillstand der Wasserstoffaufnahme (=1313 ml $H_2$, entsprechend 98% d.Th.) hydriert. Anschliessend wird das Gemisch vom Katalysator abfiltriert und die erhaltene Lösung eingedampft.

b) Der Rückstand von (a) wird in 45 ml Eisessig aufgenommen und 1 Stunde bei 50°C gerührt. Anschliessend wird eingedampft und der Rückstand mit Essigsäureäthylester/Wasser verteilt. Die organische Phase wird über $MgSO_4$ getrocknet und dann eingedampft. Der erhaltene Rückstand kristallisiert langsam durch und wird mit Diäthyläther digeriert. Es wird ein weisses Pulver erhalten.

Smp. 150—151°C. IR ($CHCl_3$) in $cm^{-1}$; 3450 (NH), 2200 (CN), 1605 (C=C).

NMR ($CDCl_3$) in ppm: 3,4 (dxq, 1H); 4,2 (m, 2H, davon 1H mit $D_2O$ austauschbar); 4,8 (q, 1H); 7,2—7,4 (m, 4H). MG: 238.

Beispiel 5:
Herstellung von 3-Cyano-4-(2',3'-dichlorphenyl)-$\Delta^2$-pyrrolin

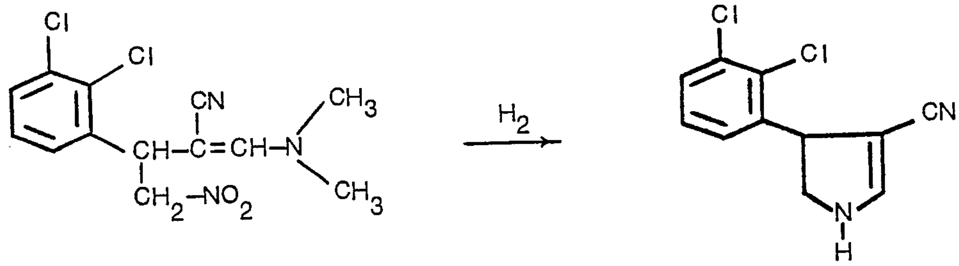


31,4 g 1-Dimethylamino-2-cyano-3-(2',3'-dichlorphenyl)-4-nitrobut-1-en werden in 400 ml Dioxan bei 100°C und 100 bar Anfangsdruck Wasserstoff in Gegenwart von 6,2 g Cobaltpolysulfid 7,5 Stunden hydriert. Nach diesem Zeitraum zeigt die Messung bei Raumtemperatur eine Wasserstoffaufnahme von 6,39 1 $H_2$ (entsprechend 95% d.Th.) an. Anschliessend wird das Gemisch filtriert, der Katalysator mit Dioxan nachgewaschen und das Filtrat eingedampft. Der Rückstand wird mit Toluol/Essigsäureäthylester (2:1) über Kieselgel chromatographiert. Es wird ein weisses Pulver erhalten.

Smp. 150°C. IR ($CHCl_3$) in $cm^{-1}$: 3450 (NH), 2200 (CN), 1605 (C=C).

NMR ($CDCl_3$) in ppm: 3,4 (dxq, 1H); 4,2 (m, 2H, davon 1H mit $D_2O$ austauschbar); 4,8 (q, 1H); 7,2—7,4 (m, 4H). MG: 238.

Beispiel 6:
Herstellung von 3-Cyano-4-(2',3'-dichlorphenyl)-pyrrol

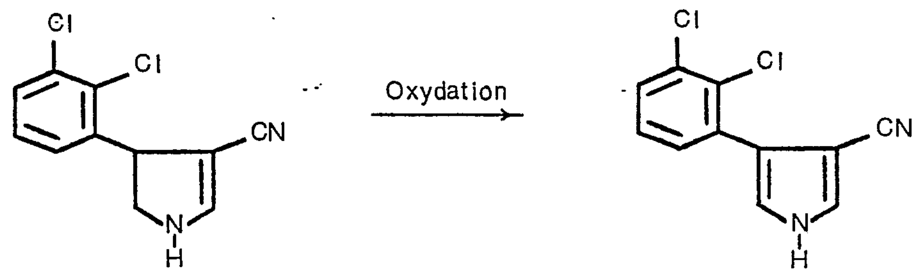


a) 1,0 g 3-Cyano-4-(2',3'-dichlorphenyl)-$\Delta^2$-pyrrolin wird in 15 ml $CHCl_3$ gelöst und mit 0,67 g Brom versetzt. Nach 15 Minuten ist dünnschichtchromatographisch kein Edukt mehr nachzuweisen. Die Reaktionslösung wird mit 1,4 ml Triäthylamin versetzt und 1 Stunde bei 40°C gerührt. Anschliessend wird das Reaktionsgemisch mit Wasser gewaschen, dann die organische Phase über $MgSO_4$ getrocknet und eingedampft. Der Rückstand wird in Toluol/Essigsäureäthylester (4:1 V/V) gelöst über Kieselgel chromatographiert. Aus dem Eluat wird das hergestellte Produkt erhalten.

Smp. 148—150°C.

b) 5,2 g 3-Cyano-4-(2',3'-dichlorphenyl)-$\Delta^2$-pyrrolin werden in 100 ml Mesitylen in Gegenwart von 0,5 g Palladium/Aktivkohle-Katalysator 14 Stunden bei 180—200°C gerührt. Anschliessend wird der Katalysator abfiltriert und das Filtrat eingedampft. Der Rückstand wird in Toluol/Essigsäureäthylester (4:1 V/V) gelöst über Kieselgel chromatographiert. Aus dem Eluat wird das hergestellte Produkt erhalten.

Smp. 149—151°C

c) 0,5 g 3-Cyano-4-(2',3'-dichlorphenyl)-$\Delta^2$-pyrrolin werden mit 1,0 g $FeCl_3$ in einem Gemisch von 12 ml Wasser/Ethanol (3:1) 24 Stunden bei Raumtemperatur unter Einleiten von Luft gerührt. Das Reaktionsgemisch wird auf Wasser gegossen und dreimal mit 10 ml Aethylacetat extrahiert. Die organische Phase wird mit Wasser gewaschen, über $MgSO_4$ getrocknet und eingedampft. Der Rückstand wird durch Verreiben mit Diäthyläther kristallisiert. Man erhält beigefarbene Kristalle.

Smp. 150—151°C.

Die einzelnen Reaktionsschritte des erfindungsgemässen Herstellungsverfahrens lassen erkennen, dass der Substituent $R_n$ im Phenylkern keinen nennenswerten Einfluss auf den Reaktionsverlauf ausübt. Auf gleiche Art lassen sich beispielsweise auch folgende Verbindungen herstellen, die als Fungizide einsetzbar sind:

3-Cyano-4-(2-chlorphenyl)-$\Delta^2$-pyrrolin,
3-Cyano-4-(2,5-dichlorphenyl)-$\Delta^2$-pyrrolin,
3-Cyano-4-(2-trifluorphenyl)-$\Delta^2$-pyrrolin,
3-Cyano-4-(2-bromophenyl)-$\Delta^2$-pyrrolin,

3-Cyano-4-(3-bromophenyl)-$\Delta^2$-pyrrolin,
3-Cyano-4-(3-fluorophenyl)-$\Delta^2$-pyrrolin,
3-Cyano-4-(3-tolyl)-$\Delta^2$-pyrrolin,
3-Cyano-4-(4-chlorphenyl)-$\Delta^2$-pyrrolin,
3-Cyano-4-(3-chlorphenyl)-$\Delta^2$-pyrrolin,
3-Cyano-4-(4-bromphenyl)-$\Delta^2$-pyrrolin,
3-Cyano-4-(4-fluorphenyl)-$\Delta^2$-pyrrolin,
3-Cyano-4-(4-tolyl)-$\Delta^2$-pyrrolin,
3-Cyano-4-(2,4-dichlorphenyl)-$\Delta^2$-pyrrolin.

Durch Oxidation sind aus solchen Verbindungen der Formel I entsprechende Pyrrol-Derivate der Formel VII herstellbar.

Formulierungsbeispiele für Wirkstoffe der Formel I
(% = Gewichtsprozent)

| 1. Emulsions-Konzentrate | a) | b) | c) |
|---|---|---|---|
| Wirkstoff aus den Beispielen 4/5 oder aus der vorstehenden Tabelle | 25% | 40% | 50% |
| Ca-Dodecylbenzolsulfonat | 5% | 8% | 6% |
| Ricinusöl-polyethylenglykolether (36 Mol Ethylenoxid) | 5% | — | — |
| Tributylphenoyl-polyethylenglykolether (30 Mol Ethylenoxid) | — | 12% | 4% |
| Cyclohexanon | — | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff aus den Beispielen 4/5 oder aus der Tabelle | 80% | 10% | 5% | 95% |
| Ethylenglykol-monomethyl-ether | 20% | — | — | — |
| Polyethylenglykol MG400 | — | 70% | — | — |
| N-Methyl-2-pyrrolidon | — | 20% | — | — |
| Epoxydiertes Kokosnussöl | — | — | 1% | 5% |
| Benzin (Siedegrenzen 160—190°C) | — | — | 94% | — |

(MG=Molekulargewicht)
Die Lösungen sind zur Anwendung in Form kleinster Tropfen geeignet.

| 3. Granulate | a) | b) |
|---|---|---|
| Wirkstoff aus den Beispielen 4/5 oder aus der Tabelle | 5% | 10% |
| Kaolin | 94% | — |
| Hochdisperse Kieselsäure | 1% | — |
| Attapulgit | — | 90% |

Der Wirkstoff wird in Methylenchlorid gelöst, auf den Träger aufgesprüht und das Lösungsmittel anschliessend im Vakuum abgedampft.

4. Stäubemittel

| | a) | b) |
|---|---|---|
| Wirkstoff aus den Beispielen 4/5 oder aus der Tabelle | 2% | 5% |
| Hochdisperse Kieselsäure | 1% | 5% |
| Talkum | 97% | — |
| Kaolin | — | 90% |

Durch inniges Vermischen auf Trägerstoffe mit dem Wirkstoff erhält man gebrauchsfertige Stäubemittel.

5. Spritzpulver

| | a) | b) | c) |
|---|---|---|---|
| Wirkstoff aus den Beispielen 4/5 oder aus der Tabelle | 25% | 50% | 75% |
| Na-Ligninsulfonat | 5% | 5% | — |
| Na-Laurylsulfat | 3% | — | 5% |
| Na-Diisobutylnaphthalinsulfonat | — | 6% | 10% |
| Octylphenolpolyethylenglykolether (7—8 Mol Ethylenoxid) | — | 2% | — |
| Hochdisperse Kieselsäure | 5% | 10% | 10% |
| Kaolin | 62% | 27% | — |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrat

| | |
|---|---|
| Wirkstoff aus den Beispielen 4/5 oder aus der Tabelle | 10% |
| Octylphenolpolyethylenglykolether (4—5 Mol Ethylenoxid) | 3% |
| Ca-Dodecylbenzolsulfonat | 3% |
| Ricinusölpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

7. Stäubemittel

| | a) | b) |
|---|---|---|
| Wirkstoff aus den Beispielen 4/5 oder aus der Tabelle | 5% | 8% |
| Talkum | 95% | — |
| Kaolin | — | 92% |

Man erhält anwendungsfertige Stäubemittel, indem der Wirkstoff mit dem Träger vermischt und auf einer geeigneten Mühle vermahlen wird.

8. Extruder-Granulat

| | |
|---|---|
| Wirkstoff aus den Beispielen 4/5 oder aus der Tabelle | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

9. Umhüllungs-Granulat

| | |
|---|---|
| Wirkstoff aus den Beispielen 4/5 oder aus der Tabelle | 3% |
| Polyethylenglykol (MG 200) | 3% |
| Kaolin (MG=Molekulargewicht) | 94% |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff aus den Beispielen 4/5 oder aus der Tabelle | 40% |
| Ethylenglykol | 10% |
| Nonylphenolpolyethylenglykolether (15 Mol Ethylenoxid) | 6% |
| N-Ligninsulfonat | 10% |
| Carboxymethylcellulose | 1% |
| 37 %ige wässrige Formaldehyd-Lösung | 0,2% |
| Silikonöl in Form einer 75 %igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele:

Beispiel 1:
Wirkung gegen Botrytis cinerea auf Bohnen Residual-protektive Wirkung

Ca. 10 cm hohe Bohnen-Pflanzen wurden mit einer aus Spritzpulver der Wirkstoffes hergestellten Spritzbrühe (0,002% Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95—100% relativer Luftfeuchtigkeit und 21°C erfolgte die Beurteilung des Pilzbefalls. Die Verbindungen aus den Beispielen 4 und 5 und aus der Tabelle hemmten nicht nur im obigen Modellversuch sondern auch im Freiland die Pilzinfektion sehr stark. Bei einer Konzentration von 0,002% erweist sich z.B. die Verbindung der Beispiele 4/5 als voll wirksam (Krankheitsbefall 0 bis 5%). Der Befall infizierter jedoch unbehandelter Bohnenpflanzen betrug 100%.

Beispiel 2:

Wirkung gegen Botrytis cinerea an Apfelfrüchten

Künstlich verletzte Aepfel wurden behandelt, indem eine aus Spritzpulver der Wirksubstanz hergestellte Spritzbrühe (0,002% Aktivsubstanz) auf die Verletzungsstellen aufgetropft wurde. Die behandelten Früchte wurden anschliessend mit einer Sporensuspension von *Botrytis cinerea* inokuliert und während einer Woche bei einer hohen Luftfeuchtigkeit und ca. 20°C inkubiert. Bei der Auswertung wurden die angefaulten Verletzungsstellen gezählt und daraus die fungizide Wirkung der Testsubstanz abgeleitet. Unter anderen hemmt die Verbindung der Beispiele 4/5 den Pilzbefall im Vergleich zu unbehandelten Kontrollfrüchten (100% Befall) vollständig.

**Patentansprüche**

1. Verbindungen der Formel I

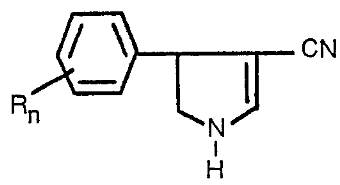

(I),

in welcher

R Halogen, $C_1$—$C_6$-Alkyl oder $C_1$—$C_6$-Haloalkyl darstellt und n 0, 1 oder 2 bedeutet.

2. Verbindungen der Formel I nach Anspruch 1, dadurch gekennzeichnet, dass R Chlor, Brom oder Methyl und n 1 oder 2 bedeuten.

3. Verbindungen der Formel I nach Anspruch 1, dadurch gekennzeichnet, dass R Chlor oder Methyl und n 2 bedeuten.

4. 3-Cyano-4-(2',3'-dichlorphenyl)-$\Delta^2$-pyrrolin.

5. Verfahren zur Herstellung der Verbindungen der Formel I

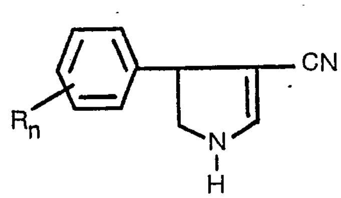

(I),

in welcher

R Halogen, $C_1$—$C_6$-Alkyl oder $C_1$—$C_6$-Haloalkyl darstellt und n 0, 1 oder 2 bedeutet, dadurch gekennzeichnet, dass man Verbindungen der Formel II

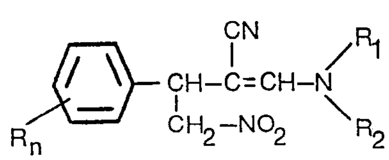

(II)

zu Verbindungen der Formel III

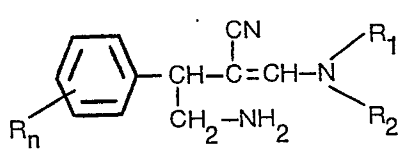

(III)

in welchen $R_1$ und $R_2$ unabhängig voneinander $C_1$—$C_4$-Alkyl, oder zusammen mit dem benachbarten Stickstoffatom Pyrrolidinyl oder Piperidinyl oder mit einem zusätzlichen Sauerstoffatom Morpholinyl bedeuten, in organischen Lösungsmitteln unter einem Wasserstoffdruck von 1 bis 150 bar in Gegenwart eines Katalysators bei Temperaturen von 0° bis 150°C reduziert und anschliessend diese bei den obengenannten Temperaturen zu Verbindungen der Formel I zyklisiert.

6. Verfahren nach Anspruch 5, dadurch gekennzeichnet, dass $R_1$ und $R_2$ Methyl bedeutet.

7. Verfahren nach Anspruch 5, dadurch gekennzeichnet, dass Reduktion und Cyclisierung in einem Reaktionsschritt erfolgen.

8. Verfahren nach Anspruch 5, dadurch gekennzeichnet, dass als Katalysator Cobaltpolysulfid oder Platin-Aktivkohle verwendet werden.

9. Verfahren nach Anspruch 5, dadurch gekennzeichnet, dass die Temperatur für die Reduktion 15° bis 110°C beträgt.

10. Verfahren nach Anspruch 5, dadurch gekennzeichnet, dass die Temperatur für den Ringschluss 50° bis 150°C beträgt.

11. Verfahren nach Anspruch 7, dadurch gekennzeichnet, dass die Reaktionstemperatur 15° bis 150°C beträgt.

12. Verfahren nach Anspruch 5 und 10, dadurch gekennzeichnet, dass der Ringschluss in schwach saurem Milieu durchgeführt wird.

13. Verfahren zur Herstellung der Verbindungen der Formel

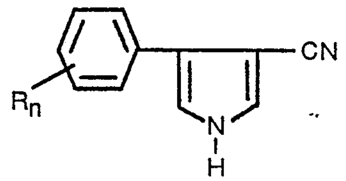

(VII),

in welcher

R Halogen, $C_1$—$C_6$-Alkyl oder $C_1$—$C_6$-Haloalkyl darstellt und n 0, 1 oder 2 bedeutet, dadurch gekennzeichnet, dass man Verbindungen der Formel I

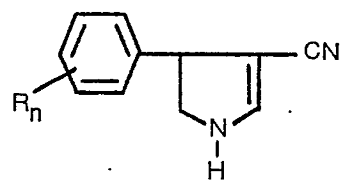

(I),

in welcher

R Halogen, $C_1$—$C_6$-Alkyl odere $C_1$—$C_6$-Haloalkyl darstellt und n 0, 1 oder 2 bedeutet, mittels Brom oder in Gegenwart eines als oxidierendes Medium wirkenden Katalysators in inerten Lösungsmitteln bei Temperaturen von 0° bis 250°C oxidiert.

14. Verfahren gemäss Anspruch 13, dadurch gekennzeichnet, dass als Katalysator Palladium/Kohle verwendet wird.

15. Verfahren gemäss Anspruch 13, dadurch gekennzeichnet, dass als Katalysator mehrwertige Metallkationen verwendet werden.

16. Verfahren gemäss Anspruch 15, dadurch gekennzeichnet, dass als Metallkation $Fe^{+++}$ eingesetzt wird.

17. Verfahren gemäss Anspruch 16, dadurch gekennzeichnet, dass $FeCl_3$ als Katalysator eingesetzt wird.

18. Verfahren gemäss Anspruch 13, dadurch gekennzeichnet, dass zur Oxidation Luft eingesetzt wird.

19. Verfahren gemäss Anspruch 15, dadurch gekennzeichnet, dass der Temperaturbereich 0° bis 90° beträgt.

20. Verbindungen der Formel II

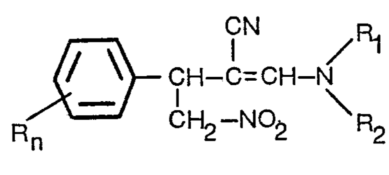

(II)

in welcher $R_1$ und $R_2$ unabhängig voneinander $C_1$—$C_4$-Alkyl, oder zusammen mit dem benachbarten Stickstoffatom Pyrrolidinyl oder Piperidinyl oder mit einem zusätzlichen Sauerstoffatom Morpholinyl, R Halogen, $C_1$—$C_6$-Alkyl oder $C_1$—$C_6$-Haloalkyl bedeuten und n 0, 1 oder 2 darstellt.

21. Verbindungen der Formel II nach Anspruch 20, dadurch gekennzeichnet, dass $R_1$ und $R_2$ unabhängig voneinander Methyl bedeuten.

22. Mittel zur Bekämpfung oder Verhütung eines Befalls durch Mikroorganismen, dadurch gekennzeichnet, dass es als aktive Komponente mindestens eine Verbindung der Formel I gemäss Anspruch 1 enthält.

23. Mittel gemäss Anspruch 22, dadurch gekennzeichnet, dass es als aktive Komponente mindestens eine Verbindung gemäss Anspruch 2 enthält.

24. Mittel zur Bekämpfung von Mikroorganismen gemäss Anspruch 23, dadurch gekennzeichnet, dass es als aktive Komponente mindestens eine Verbindung gemäss Anspruch 3 enthält.

25. Mittel zur Bekämpfung von Mikroorganismen gemäss Anspruch 22, dadurch gekennzeichnet, dass es als aktive Komponente die Verbindung gemäss Anspruch 4 enthält.

26. Mittel nach einem der Ansprüche 22 bis 25, dadurch gekennzeichnet, dass es 0,1 bis 99% eines Wirkstoffs der Formel I, 99,9 bis 1% eines festen oder flüssigen Zusatzstoffes und 0 bis 25% eines Tensides enthält.

27. Verfahren zur Herstellung eines agrochemischen Mittels, dadurch gekennzeichnet, dass mindestens eine Verbindung der Formel I gemäss einem der Ansprüche 1 bis 4 mit geeigneten festen oder flüssigen Zusatzstoffen und Tensiden innig vermischt wird.

28. Verfahren zur Bekämpfung oder Verhütung eines Befalls von Kulturpflanzen durch phytopathogene Mikroorganismen, dadurch gekennzeichnet, dass man eine Verbindung der Formel I auf die Pflanze oder deren Standort appliziert.

29. Verwendung von Verbindungen der Formel I zur Bekämpfung von Pflanzenkrankheiten, die auf Mikroorganismen zurückzuführen sind.

30. Verwendung gemäss Anspruch 29, dadurch gekennzeichnet, dass es sich bei den Mikroorganismen um phytopathogene Pilze handelt.

**Revendications**

1. Composés de formule I

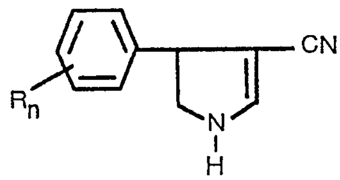

(I),

dans laquelle
R représente un halogène, un groupe alkyle en $C_1$—$C_6$ ou halogénoalkyle en $C_1$—$C_6$, et n est égal à 0, 1 ou 2.

2. Composé de formule I selon la revendication 1, caractérisés en ce que R représente le chlore, le brome ou un groupe méthyle et n est égal à 1 ou 2.

3. Composés de formule I selon la revendication 1, caractérisés en ce que R représente le chlore ou un groupe méthyle et n est égal à 2.

4. La 3-cyano-4-(2',3'-dichlorophényl)-$\Delta^2$-pyrroline.

5. Procédé de préparation des composés de formule I

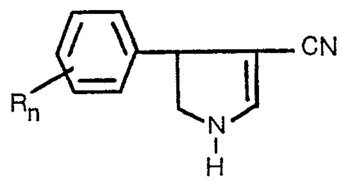

(I),

dans laquelle
R représente un halogène, un groupe alkyle en $C_1$—$C_6$ ou halogénoalkyle en $C_1$—$C_6$ et n est égal à 0, 1 ou 2, caractérisé en ce que l'on réduit des composés de formule II

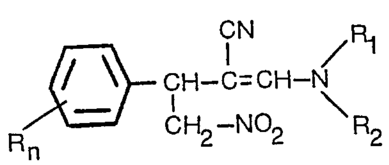

(II)

en composés de formule III

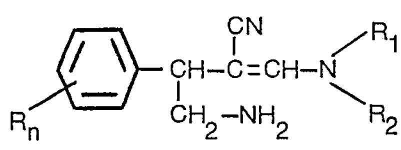

(III)

dans lesquels $R_1$ et $R_2$ représentent chacun, indépendamment l'un de l'autre, un groupe alkyle en $C_1$—$C_4$ ou forment ensemble et avec l'atome d'azote voisin un groupe pyrrolidinyle ou pipéridinyle, ou avec un atome

supplémentaire d'oxygène, un groupe morpholidinyle, dans des solvants organiques, sous une pression d'hydrogène de 1 à 150 bars, en présence d'un catalyseur, à des températures de 0 à 150°C, puis on cyclise ces composés, aux mêmes températures, en composés de formule I.

6. Procédé selon la revendication 5, caractérisé en ce que $R_1$ et $R_2$ représentent des groupes méthyle.

7. Procédé selon la revendication 5, caractérisé en ce que la réduction et la cylisation sont effectuées en un seul stade de réaction.

8. Procédé selon la revendication 5, caractérisé en ce que l'on utilise en tant que catalyseur du polysulfure de cobalt ou du platine sur charbon actif.

9. Procédé selon la revendication 5, caractérisé en ce que la température de réduction est de 15 à 110°C.

10. Procédé selon la revendication 5, caractérisé en ce que la température de cyclisation est de 50 à 150°C.

11. Procédé selon la revendication 7, caractérisé en ce que la température de réaction est de 15 à 150°C.

12. Procédé selon les revendications 5 et 10, caractérisé en ce que la cyclisation est effectuée en mileu légèrement acide.

13. Procédé de préparation des composés de formule

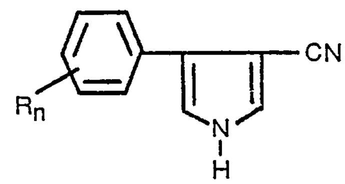

(VII),

dans laquelle

R représente un halogène, un groupe alkyle en $C_1$—$C_6$ ou halogénoalkyle en $C_1$—$C_6$ et n est égal à 0, 1 ou 2, caractérisé en ce que l'on oxyde des composés de formule I

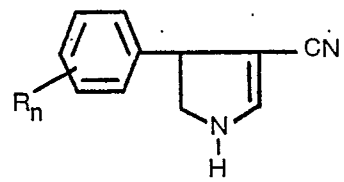

(I),

dans laquelle

R représente un halogène, un groupe alkyle en $C_1$—$C_6$ ou halogénoalkyle en $C_1$—$C_6$ et n est égal à 0, 1 ou 2, à l'aide du brome ou en présence d'un catalyseur faisant fonction d'agent oxydant, dans des solvants inertes, à des températures de 0 à 250°C.

14. Procédé selon la revendication 13, caractérisé en ce que le catalyseur utilisé est le palladium sur charbon.

15. Procédé selon la revendication 13, caractérisé en ce que l'on utilise en tant que catalyseurs des cations métalliques polyvalents.

16. Procédé selon la revendication 15, caractérisé en ce que l'on utilise en tant que cation métallique $Fe^{+++}$.

17. Procédé selon la revendication 16, caractérisé en ce que l'on utilise en tant que catalyseur $FeCl_3$.

18. Procédé selon la revendication 13, caractérisé en ce que, pour l'oxydation, on utilise de l'air.

19. Procédé selon la revendication 15, caractérisé en ce que l'on opère dans un intervalle de température de 0 à 90°C.

20. Composés de formule II

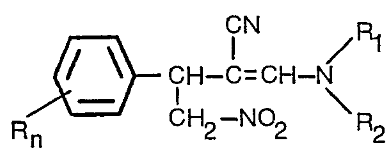

(II)

dans laquelle $R_1$ et $R_2$ représentent chacun, indépendamment l'un de l'autre, un groupe alkyle en $C_1$—$C_4$ ou forment ensemble et avec l'atome d'azote voisin un groupe pyrrolidinyle ou pipéridinyle, ou, avec un atome supplémentaire d'oxygène, un groupe morpholinyle, R représente un halogène, un groupe alkyle en $C_1$—$C_6$ ou halogénoalkyle en $C_1$—$C_6$ et n est égal à 0, 1 ou 2.

21. Composés de formule II selon la revendication 20, caractérisés en ce que $R_1$ et $R_2$ représentent chacun, indépendamment l'un de l'autre, un groupe méthyle.

22. Produit pour combattre ou prévenir une attaque par des microorganismes, caractérisé en ce qu'il contient au moins un composant actif consistant en un composé de formule I selon la revendication 1.

23. Produit selon la revendication 22, caractérisé en ce qu'il contient au moins un composant actif consistant en un composé selon la revendication 2.

24. Produit pour combattre les microorganismes selon la revendication 23, caractérisé en ce qu'il contient au moins un composant actif consistant en un composé selon la revendication 3.

25. Produit pour combattre les microorganismes selon la revendication 22, caractérisé en ce qu'il contient en tant que composant actif le composé de la revendication 4.

26. Produit selon l'une des revendications 22 à 25, caractérisé en ce qu'il contient de 0,1 à 99% d'une substance active de formule I, de 99,9 à 1% d'un additif solide ou liquide et de 0 à 25% d'un agent tensioactif.

27. Procédé de préparation d'un produit agrochimique, caractérisé en ce que l'on mélange intimement au moins un composé de formule I selon l'une des revendications 1 à 4 avec des additifs et agents tensioactifs solides ou liquides appropriés.

28. Procédé pour combattre ou prévenir une attaque de végétaux cultivés par des microorganismes phytopathogènes, caractérisé en ce que l'on applique un composé de formule I sur la plante ou son biotope.

29. Utilisation des composés de formule I pour la lutte contre les maladies des végétaux qui sont dues à des microorganismes.

30. Utilisation selon la revendication 29, caractérisée en ce que les microorganismes sont des mycètes phytophathogènes.

## Claims

1. Compounds of the formula I

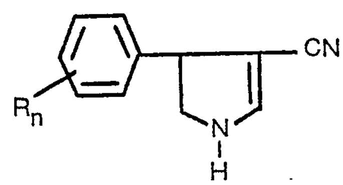

(I)

wherein
R is halogen, $C_1$—$C_6$alkyl or $C_1$—$C_6$haloalkyl, and
n is 0, 1 or 2.

2. Compounds of the formula I according to claim 1, wherein R is chlorine, bromine or methyl, and n is 1 or 2.

3. Compounds of the formula I according to claim 1, wherein R is chlorine or methyl, and n is 2.

4. 3-cyano-4-(2',3'-dichlorophenyl)-$\Delta^2$-pyrroline.

5. A process for producing compounds of the formula I

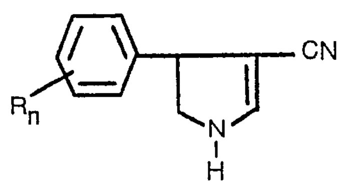

(I)

wherein
R is halogen, $C_1$—$C_6$alkyl or $C_1$—$C_6$haloalkyl, and
n is 0, 1 or 2, which process comprises reducing compounds of the formula II

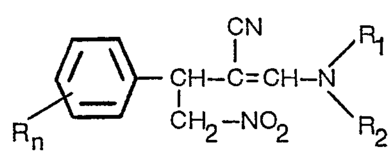

(II)

to compounds of the formula III

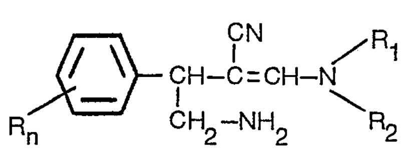

(III)

in which $R_1$ and $R_2$ independently or one another are each $C_1$—$C_4$alkyl, or together with the adjacent nitrogen atom are pyrrolidinyl or piperidinyl, or with an additional oxygen atom are morpholinyl, in organic

solvents under a hydrogen pressure of 1 to 150 bar, in the presence of a catalyst, at temperatures of 0° to 150°C; and subsequently cyclising those compounds, at the aforementioned temperatures, to compounds of the formula I.

6. A process according to claim 5, wherein $R_1$ and $R_2$ are methyl.

7. A process according to claim 5, wherein reduction and cyclisation are performed in one reaction step.

8. A process according to claim 5, wherein the catalyst used is cobalt polysulfide or platinum-active charcoal.

9. A process according to claim 5, wherein the temperature for the reduction reaction is 15° to 110°C.

10. A process according to claim 5, wherein the temperature for the ring closure reaction is 50° to 150°C.

11. A process according to claim 7, wherein the reaction temperature is 15° to 150°C.

12. A process according to claims 5 and 10, wherein the ring closure reaction is performed in a weakly acidic medium.

13. A process for producing compounds of the formula

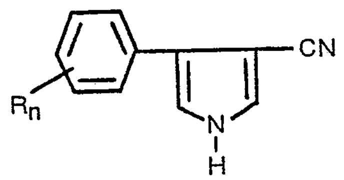

(VII)

wherein

R is halogen, $C_1$—$C_6$alkyl or $C_1$—$C_6$haloalkyl, and

n is 0, 1 or 2,

which process comprises oxidising compounds of the formula I

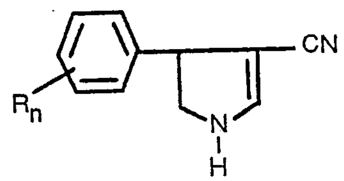

(I)

wherein

R is halogen, $C_1$—$C_6$alkyl or $C_1$—$C_6$haloalkyl, and

n is 0, 1 or 2,

by means of bromine, or in the presence of a catalyst acting as the oxidising medium, in inert solvents at temperatures of 0° to 250°C.

14. A process according to claim 13, wherein the catalyst used is palladium/charcoal.

15. A process according to claim 13, wherein the catalyst used is a polyvalent metal cation.

16. A process according to claim 15, wherein the metal cation used is $Fe^{+++}$.

17. A process according to claim 16, wherein the catalyst used is $FeCl_3$.

18. A process according to claim 13, wherein air is used for the oxidation process.

19. A process according to claim 15, wherein the temperature range is 0° to 90°.

20. Compounds of the formula II

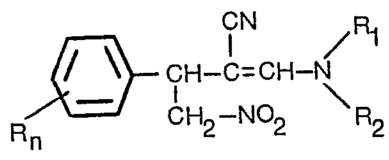

(II)

in which $R_1$ and $R_2$ independently of one another are each $C_1$—$C_4$alkyl, or together with the adjacent nitrogen atom are pyrrolidinyl or piperidinyl, or with an additional oxygen atom are morpholinyl, R is halogen, $C_1$—$C_6$alkyl or $C_1$—$C_6$haloalkyl, and n is 0, 1 or 2.

21. Compounds of the formula II according to claim 20, wherein $R_1$ and $R_2$ independently of one another are each methyl.

22. A composition for controlling or preventing an infestation by microorganisms, which composition contains, as active component, at least one compound of the formula I according to claim 1.

23. A composition according to claim 22, which contains, as active component, at least one compound according to claim 2.

24. A composition for controlling microorganisms, according to claim 23, which contains, as active component, at least one compound according to claim 3.

25. A composition for controlling microorganisms, according to claim 22, which contains, as active component, a compound according to claim 4.

26. A composition according to any one of claims 22 to 25, which contains 0.1 to 99% of an active ingredient of the formula I, 99.9 to 1% of a solid or liquid additive, and 0 to 25% of a surfactant (tenside).

27. A process for producing an agrochemical composition, which process comprises intimately mixing at least one compound of the formula I according to any one of claims 1 to 4 with suitable solid or liquid additives and surfactants.

28. A process for controlling or preventing an infestation of cultivated plants by phytopathogenic microorganisms, which process comprises applying a compound of the formula I to the plants or to the locus thereof.

29. The use of compounds of the formula I for controlling plant diseases that are attributable to microorganisms.

30. The use according to claim 29, wherein the microorganisms are phytopathogenic fungi.